# EUROPÄISCHE PATENTSCHRIFT

(11) **EP 4 317 396 B1**
(45) Veröffentlichungstag und Bekanntmachung des Hinweises auf die Patenterteilung: **12.02.2025**
(21) Anmeldenummer: 22188086.7
(22) Anmeldetag: 01.08.2022
(51) Int. Cl.: C12M 1/00, C12M 3/00, C12M 1/34, C12N 13/00

(54) **SINGLE-USE BAGHALTER ZUR VERWENDUNG MIT PLATINEN-EINBAUTEN**
SINGLE-USE BAGHOLDER FOR USE WITH PLATINUM INSERTS
PORTE-SAC À USAGE UNIQUE DESTINÉ À ÊTRE UTILISÉ AVEC DES INSTALLATIONS DE CARTES DE CIRCUIT IMPRIMÉ

(43) Veröffentlichungstag der Anmeldung: 07.02.2024
(73) Patentinhaber: Sartorius Stedim Systems GmbH, 34302 Guxhagen (DE)
(72) Erfinder: SCHMID, Hannes, 34587 Felsberg (DE)
(74) Vertreter: Novagraaf International SA

(56) Entgegenhaltungen:
- EP-A1- 3 553 161
- WO-A1-2017/157404
- WO-A1-2019/034792
- DE-A1- 102010 018 678
- DE-A1- 102015 007 061
- DE-A1- 102017 008 769
- DE-U1- 202007 013 406
- US-A1- 2011 310 696

## Beschreibung

Die Erfindung betrifft eine Vorrichtung und ein Verfahren zum Aufnehmen eines Einwegbehälters.

Bioreaktoren und Pallettanks dienen als Vorrichtung zur Aufnahme, Lagerung und/oder Kultivierung von biologischen Medien wie z.B. Fluiden und/oder Zellkulturen. Biologische Medien können in Einwegbehältern bereitgestellt werden, die ein Volumen von wenigen Litern bis zu mehreren tausend Litern umfassen können. Die biologischen Medien werden innerhalb eines Einwegbehälters in dem Bioreaktor eingebracht, in dem sie über einen vorbestimmten Zeitraum von üblicherweise mehreren Stunden Dauer auf einer vorbestimmten Temperatur temperiert werden. Weiterhin können in einem solchen Bioreaktor unterschiedliche Untersuchungen an dem biologischen Medium vorgenommen werden. Die Handhabung eines Bioreaktors kann unter Reinraumbedingungen erfolgen, so dass besondere hohe Anforderungen an die Qualitätssicherung am Bioreaktor gestellt werden.

Sogenannte Single-Use-Prozesse sind in der pharmazeutischen Industrie mittlerweile weit verbreitet. Als Einwegbehälter können in Single-Use-Prozessen insbesondere Einwegbeutel verwendet werden. Sowohl im Upstream, als auch im Downstream erweist sich der Einsatz von Einwegbehältern als zeit- und kostensparend und prozesstechnisch sicher. Während es mittlerweile Erfahrungen mit Zellkulturprozessen und mikrobiellen Kulturen gibt, konnten bislang Prozesse mit phototrophen Organismen (Algen, Pflanzenzellkulturen) nur eingeschränkt verwirklicht werden. Dies gilt insbesondere für einen Produktionsmaßstab ab 50 Litern.

WO 2017/157404 offenbart einen Aufnahmebehälter zur Aufnahme eines transparenten Einwegbehälters, der als flexibler Beutel gestaltet ist. An der Innenseite des zylindrischen Aufnahmebehälters ist eine Beleuchtungsvorrichtung angeordnet.

Die Aufnahmebehälterwand wird von einer Temperiereinheit temperiert. Die Beleuchtungsvorrichtung ist so im Behälterinnenraum angeordnet, dass sie im Wärmeaustausch mit der temperierten Aufnahmebehälterwand steht. Dies ermöglicht eine Wärmeabfuhr der Beleuchtungswärme, die bei Betrieb der Beleuchtungsvorrichtung entstehen kann.

Aufgabe der Erfindung ist es daher, die Kultivierung von phototrophen Organismen insbesondere in einem größeren Produktionsmaßstab zu vereinfachen, insbesondere die Kontrolle von Produktionsparametern zu verbessern. Diese Aufgabe wird durch eine Vorrichtung und ein Verfahren gemäß den unabhängigen Ansprüchen gelöst. Bevorzugte Ausführungsformen sind Gegenstand der abhängigen Ansprüche.

Ein Aspekt betrifft damit eine Vorrichtung zur Aufnahme eines Einwegbehälters, umfassend einen Aufnahmebehälter mit einer Aufnahmebehälterinnenwand, die einen Behälterinnenraum des Aufnahmebehälters zum Aufnehmen des Einwegbehälters begrenzt, wobei in der Aufnahmebehälterinnenwand eine Vielzahl von Nuten bzw. Kanälen zur Aufnahme elektrischer Funktionselemente vorgesehen sind. Als solche elektrische Funktionselemente können Temperierelemente, insbesondere aber Leuchteinheiten (z.B. LEDs) eingesetzt werden.

Die Vorrichtung kann als ein Bioreaktor und/oder Pallettank ausgebildet sein, der zur Aufnahme von Einwegbehältern mit einem Volumen von ca. 1 Liter bis ca. 5000 Litern ausgebildet sein kann, bevorzugt mit einem Volumen von ca. 50 Litern bis ca. 2000 Litern, besonders bevorzugt zumindest ca. 500 Litern. Die Vorrichtung kann als ein System mit mehreren Komponenten ausgebildet sein, insbesondere mit oder ohne einem Einwegbehälter als zugehörige Komponente. Die Vorrichtung kann insbesondere zum Aufnehmen von Einwegbehältern ausgebildet sein, in denen sich ein biologisches Medium wie ein Fluid befindet, das über einen vorbestimmten Zeitraum im System gelagert, temperiert, kultiviert und/oder anders untersucht werden soll. Das biologische Medium kann als eine Zellkultur ausgebildet sein und/oder phototrophe Organismen aufweisen.

Der Einwegbehälter kann als ein Einwegbeutel mit flexiblen Beutelwänden ausgebildet sein, insbesondere mit transparenten Kunststoffwänden. Der Einwegbehälter kann in seinem Inneren Rührkomponenten aufweisen, die an eine Rührvorrichtung der Vorrichtung gekoppelt werden können, um so ein Vermischen des Inhalts des Einwegbehälters zu ermöglichen.

Der Aufnahmebehälter der Vorrichtung stellt den Behälterinnenraum bereit, der zum Aufnehmen des Einwegbehälters ausgebildet ist. Dabei kann der Behälterinnenraum zum Aufnehmen eines vorbestimmbaren Typs eines Einwegbehälters ausgebildet sein, also zum Beispiel eines Einwegbehälters eines vorbestimmbaren Herstellers und/oder mit einem vorbestimmbaren Füllvolumen. Die Aufnahmebehälterinnenwand bzw. -wände definieren dabei den Behälterinnenraum. Dabei müssen die Aufnahmebehälterinnenwände den Behälterinnenraum nicht vollständig umgeben und/oder umgrenzen. So kann der Aufnahmebehälter zum Beispiel eine Rühröffnung aufweisen, durch die eine Rührvorrichtung an den im Behälterinnenraum angeordneten Einwegbehälter anschließbar ist. Eine solche Rührvorrichtung ist bevorzugt am oberen Ende des Aufnahmebehälters ausgebildet. Der Aufnahmebehälter kann somit insbesondere deckellos und/oder oben offen ausgebildet sein.

Die Begriffe "oben", "unten", "seitlich", "vertikal", "horizontal", "Höhe", "lateral" etc. beziehen sich im Rahmen dieser Erfindung auf das Bezugssystem der Erde, in dem das System bzw. die Vorrichtung in einer Betriebsstellung angeordnet ist.

Der Einwegbehälter wird bevorzugt so in den Aufnahmebehälter eingebracht, dass er auf einem Boden des Aufnahmebehälters aufliegt und in direktem, physikalischen Kontakt mit den Aufnahmebehälterinnenwänden des Aufnahmebehälters steht, insbesondere in Kontakt mit dem Boden des Aufnahmebehälters und/oder den an den Boden angrenzenden Aufnahmebehälterinnenwänden. Der Aufnahmebehälter kann zumindest eine offenbare und schließbare Tür aufweisen, durch die der Einwegbehälter in den Behälterinnenraum eingebracht werden kann.

Durch die Möglichkeit, elektrische Funktionselemente in Nuten in der Aufnahmebehälterinnenwand aufzunehmen wird deren thermische Kopplung zur Aufnahmebehälterinnenwand verbessert und gleichzeitig bleibt der Behälterinnenraum unbeeinträchtigt oder weniger beeinträchtigt von vorragenden Funktionselementen, die auch den Einwegbehälter beeinträchtigen oder beschädigen könnten.

Besonders bevorzugt sind in der Vielzahl von Nuten zumindest teilweise Leuchteinheiten zum Beleuchten des Behälterinnenraums montierbar oder montiert sind. Die Funktionselemente sind oder umfassen in diesem Fall somit vorzugsweise Leuchteinheiten. Insbesondere könnten alle Nuten ausgelegt sein, Leuchteinheiten zum Beleuchten des Behälterinnenraums aufzunehmen. In einer Ausführungsform könnten sogar in allen Nuten Leuchteinheiten montiert sein. In einer anderen bevorzugten Ausführungsform sind oder werden nur in einigen der Nuten Leuchteinheiten montiert oder montierbar, während andere Nuten für andere Funktionselemente vorgesehen sind bzw. genutzt werden also insbesondere mit anderen Funktionselemente bestückbar oder bestückt sind.

Die in den Nuten angeordneten Leuchteinheiten sind vorzugsweise so angeordnet, dass sie die Lichtleistung zumindest überwiegend in Richtung zum Behälterinnenraum also an den Einwegbehälter bzw. das darin enthaltende Medium abgeben. In der Betriebsstellung bzw. -position der Vorrichtung ist ein Einwegbehälter in die Vorrichtung eingebracht. Der Einwegbehälter muss kein Bestandteil der Vorrichtung sein. Der Einwegbehälter kann jedoch als ein Bestandteil der Vorrichtung ausgebildet sein. Die Leuchteinheiten können so im Behälterinnenraum angeordnet sein, dass sie im Wärmeaustausch mit der vorzugsweise temperierten Aufnahmebehälterinnenwand stehen. Dabei können vorzugsweise mittels einer später noch beschriebenen Temperiereinheit insbesondere die Leuchteinheiten temperiert, z.B. gekühlt werden. Dies ermöglicht eine Wärmeabfuhr einer Abwärme, die bei Betrieb der Leuchteinheiten entstehen kann. Hierbei benötigen die Leuchteinheiten keine eigene Kühlung, sondern können mittels der Temperiereinheit der Vorrichtung temperiert bzw. gekühlt werden.

Die Vorrichtung ist insbesondere zur Aufnahme von Einwegbehältern mit phototrophen Organismen oder Zellkulturen geeignet. Mittels des von den Leuchteinheiten abgestrahlten Lichts kann das im Einwegbehälter befindliche Medium bestrahlt werden. Hierbei sind die Wände des Einwegbehälters bevorzugt transparent für zumindest die Lichtwellenlänge(n) ausgebildet, in der die Leuchteinheiten Licht in den Behälterinnenraum abstrahlen. Somit betrifft die Erfindung in einem weiteren Aspekt die Verwendung einer erfindungsgemäßen Vorrichtung zur Prozessierung von phototrophen Organismen. Vorzugsweise wird die Vorrichtung für die Kultivierung von Moosen in einem Prozess zur Herstellung von alpha-Galactosidase eingesetzt.

Im Rahmen dieser Erfindung bedeutet der Begriff "Licht" nicht notwendigerweise sichtbares Licht, sondern kann elektromagnetische Strahlung einer Wellenlänge von etwa 50 nm bis etwa 50 µm betreffen. Der Begriff schließt somit insbesondere ultraviolettes und infrarotes Licht mit ein. Die Wellenlänge bzw. der Wellenlängenbereich des von den Leuchteinheiten abgestrahlten Lichts kann auf bestimmte Bedürfnisse des Mediums im Einwegbehälter abgestimmt sein. Die Leuchteinheiten können jeweils ein oder mehrere Lichtquellen aufweisen, die so ausgerichtet sind, dass sie Licht in Richtung von der Aufnahmebehälterinnenwand hin zum Behälterinnenraum abstrahlen, insbesondere in einer Richtung hin zu dem Raumgebiet, in dem in der Betriebsstellung bzw. -position der Einwegbehälter angeordnet ist, z.B. hin zu einem Mittelpunkt und/oder einer Mittelachse des Behälterinnenraums.

Die Vorrichtung kann weiterhin eine Steuerung aufweisen, die zur Steuerung der Lichtabgabe durch die Leuchteinheiten dient. Die Leuchteinheiten können z.B. modulierte und/oder nicht modulierte elektromagnetische Strahlung abstrahlen, oder auch kohärente elektromagnetische Strahlung wie z.B. Laserlicht. Die Leuchteinheiten können vorzugsweise so angesteuert werden, dass sie während ansteuerbaren Zeiträumen eine ansteuerbare Lichtmenge abstrahlen. Dabei können die Leuchteinheiten insbesondere auch abgeschaltet werden. Gemäß einer Ausführungsform weist eine (bzw. jede) Leuchteinheit eine Vielzahl von Lichtquellen auf, die innerhalb derselben Nut angeordnet sind. Die Vielzahl von Lichtquellen einer Leuchteinheit werden dabei vorzugsweise von einem gemeinsamen Träger getragen, über den die jeweilige Leuchteinheit innerhalb der Nut an der Aufnahmebehälterinnenwand angeordnet ist. Als Träger kann ein Substrat dienen, insbesondere ein zumindest stellenweise wärmeleitfähiges Substrat, wodurch eine an der oder den Lichtquellen entwickelte Wärme sehr effizient an die Aufnahmebehälterinnenwand abgeführt werden kann. Wie später noch genauer beschrieben wird, ist es besonders bevorzugt, wenn eine Temperierung auf der Außenseite der Aufnahmebehälterinnenwand mittels einer Temperiereinheit (z.B. ein Temperiermedium) erfolgt, das die Aufnahmebehälterinnenwand an der Außenseite temperiert.

In einer Ausführungsform sind die Leuchteinheiten in den Nuten zumindest teilweise durch ein (transluzentes oder transparentes) Gussmaterial bedeckt oder eingegossen (z.B. eine transluzente oder transparente Harz- bzw. Lackschicht). Dieses Gussmaterial unterstützt dabei eine mechanisch stabile Befestigung der jeweiligen Leuchteinheit in der entsprechenden Nut und schützt gleichzeitig die Leuchteinheiten und den Einwegbehälter vor gegenseitiger Beschädigung. Besonders bevorzugt erlaubt es eine zumindest teilweise Brechungsindexanpassung für einzustrahlendes Licht aus der Leuchteinheit in den Einwegbehälter, wodurch ein möglichst hoher Anteil des Lichts aus der Leuchteinheit (insbesondere aus den Lichtquellen) in den Einwegbehälter homogen verteilt eingekoppelt werden kann. Als Gussmaterial kann ein Epoxidharz verwendet werden. Die Harzschicht und/oder Lackschicht ist insbesondere transparent für diejenige oder diejenigen Wellenlänge(n) ausgebildet, die von den Leuchteinheiten abgestrahlt werden. Besonders bevorzugt wird durch das Gussmaterial eine hermetische Abdichtung (IP 65) der elektrischen Funktionseinheiten (und ihrer unmittelbaren elektrischen Anschlüsse) in den Nuten gegenüber der Umgebung erreicht.

In einer Ausführungsform sind die Nuten mit den darin montierten Leuchteinheiten zum Behälterinnenraum hin mittels (transluzenter oder transparenter) Nutabdeckungen derart verschlossen, dass Innenflächen der (transluzenten bzw. transparenten) Nutabdeckungen zusammen mit zwischen den Nuten gebildeten, insbesondere an die Nuten angrenzenden, Innenflächen der Aufnahmebehälterinnenwand stetige Flächen, also insbesondere ohne Stufen, bilden. Die dadurch entstehende stetige Aufnahmebehälterinnenfläche weist dabei vorzugsweise keine (insbesondere konvexe) Kanten auf. Dies bewirkt sogar mehrere Vorteile. Zum einen ist der Aufnahmebehälter auf dessen Innenseite leicht und gründlich zu reinigen. Weiterhin werden dadurch mögliche mechanische Beschädigungen oder Belastungen des Einwegbehälters vermieden oder reduziert.

Außerdem wird dadurch ein durchgängig guter Kontakt zwischen dem Einwegbehälter (und damit dem darin enthaltenen Medium) und dem Aufnahmebehälter erreicht. Dieser durchgängig gute, homogene Kontakt verbessert sowohl die thermische als auch die optische Ankopplung des Einwegbehälters (und damit dessen Inhalts) an die Aufnahmebehälter. Dies führt zu einer verbesserten thermischen Leistungsübertragung (z.B. Kühl- und/oder Heizleistung) und somit zu einer verlässlicheren Temperaturkontrolle selbst für große Prozessvolumina. Insbesondere werden auch lokale Temperaturspitzen vermieden, selbst wenn Leuchteinheiten in den Nuten lokal Wärmeleistung einbringen. Die gute optische Ankopplung stellt eine möglichst hohe räumliche und spektrale Homogenität einer Beleuchtung eines im Einwegbehälter enthaltenen Mediums durch die in den Nuten enthaltenen Leuchteinheiten sicher. Auch dies wiederum verbessert die Prozessqualität und -effizient vor allem auch für große Prozessvolumina.

Besonders wirkungsvoll werden diese oder einige dieser Vorteile erreicht, wenn in der jeweiligen Nut unter der Nutabdeckung, insbesondere zwischen der Leuchteinheit und der Nutabdeckung, vorzugsweise unmittelbar unterhalb der Nutabdeckung, ein Spalt (Luftspalt) gebildet wird. Die Nutabdeckungen werden vorzugsweise als Scheiben mit im Wesentlichen homogener Dicke ausgebildet. Anders als ohne solche Nutabdeckungen kann es bei Verwendung von Nutabdeckungen vorteilhaft sein, die Nuten entweder gar nicht durch ein bereits beschriebenes Gussmaterial zu füllen oder zumindest zwischen dem Gussmaterial und der Nutabdeckung einen solchen Spalt vorzusehen. Es wird also vorzugsweise innerhalb der Nuten jeweils zumindest zwischen der Leuchteinheit und der jeweiligen Nutabdeckung ein Luftspalt ausgebildet, welcher insbesondere groß genug ist, um Interferenzen der von den Leuchteinheiten erzeugten Strahlung innerhalb des Luftspaltes durch die den Luftspalt begrenzenden Flächen gering zu halten. Beispielsweise kann ein Luftspalt mit einer Dicke im Bereich von etwa 0,5 mm bis etwa 5 mm vorgesehen werden. Die Luftspalte bilden einen kontrollierbaren und stabilen optischen Übergang zwischen den Leuchteinheiten und den Nutabdeckungen. So stellte sich heraus, dass sich bei einem direkten (mechanischen) Kontakt zwischen den Nutabdeckungen und den Leuchteinheiten (oder einem die Leuchteinheiten bedeckenden Gussmaterial) die optische Qualität (insbesondere die Intensität und Homogenität) des eingestrahlten Lichts mit der Zeit verändert, insbesondere verschlechtert. Dies wurde im Rahmen dieser Entwicklung einem schlecht kontrollierbaren Ablösungseffekt zwischen den Leuchteinheiten (oder einem Gussmaterial) und den Nutabdeckungen, z.B. aufgrund thermischer und/oder mechanischer Belastungen, zugeschrieben, welcher durch das Vorsehen eines Luftspaltes vermieden wird.

Vorzugsweise weist jede der Vielzahl von Nuten einen beidseitig zweistufigen Querschnitt senkrecht zu einer Längsachse der jeweiligen Nut derart auf, dass beiderseits eines zentralen Nutkanals jeweils eine Auflagefläche um eine erste Stufe relativ zur Innenfläche der Aufnahmebehälterinnenwand und ein Nutengrund des zentralen Nutkanals um eine zweite Stufe relativ zu den beiden Auflageflächen zurückversetzt sind. Mit anderen Worten umfasst jede Nut neben einem zentralen Nutkanal (zur Aufnahme der zumindest einen Leuchteinheit) beidseitig jeweils eine Nutschulter. Die von den beidseitigen Nutschultern gebildeten Auflageflächen dienen insbesondere zum Aufnehmen der Nutabdeckung. Dazu weist insbesondere die erste Stufe eine Höhe auf, die im Wesentlichen (d.h. abgesehen von einer eventuellen Dicke einer zusätzlichen Klebstoffschicht) einer Dicke der jeweiligen Nutabdeckung entspricht. Die zweite Stufe weist dabei insbesondere eine Höhe auf, die ausreicht, um die (jeweilige) Leuchteinheit innerhalb des zentralen Nutkanals (unter der Nutabdeckung) aufnehmen zu können.

Je nach Ausführungsform liegt die Höhe der ersten Stufe beispielsweise in einem Bereich von etwa 0,5 mm bis etwa 5 mm, vorzugsweise in einem Bereich von etwa 1 mm bis etwa 3 mm. Alternativ oder zusätzlich liegt die Höhe der zweiten Stufe beispielsweise in einem Bereich von etwa 2 mm bis etwa 15 mm, vorzugsweise in einem Bereich von etwa 2 mm bis etwa 10 mm, noch mehr bevorzugt in einem Bereich von etwa 3 mm bis etwa 5 mm. Alternativ oder zusätzlich liegt eine Breite der jeweiligen Auflagefläche (Schulterbreite) beispielsweise in einem Bereich von etwa 1 mm bis etwa 5 mm, vorzugsweise in Bereich von etwa 1 mm bis etwa 3 mm. Alternativ oder zusätzlich liegt eine Breite des zentralen Nutkanals (senkrecht zu dessen Längserstreckung) beispielsweise in einem Bereich von etwa 5 mm bis etwa 30 mm, vorzugsweise in einem Bereich von etwa 10 mm bis etwa 20 mm. Damit lassen sich beispielsweise LED-Streifen gut in den Nuten verlegen. Somit umfassen die in den Nuten montierbaren oder montierten Leuchteinheiten insbesondere LEDs als Lichtquellen. Dadurch kann sowohl modulierte als auch unmodulierte Strahlung abgegeben werden. LEDs eignen sich als besonders effiziente Lichtquellen, da LEDs in Relation zur abgegebenen Lichtmenge relativ wenig Wärme entwickeln, wodurch die Wärmeentwicklung reduziert werden kann und zugleich die abgestrahlte Lichtleistung erhöht werden kann. Weiterhin können LEDs mit geringer Bauhöhe ausgebildet sein, so dass diese Lichtquellen bereits bei vergleichsweise geringer Nuttiefe vollständig innerhalb der Aufnahmebehälterinnenwand aufgenommen werden können.

In einer Ausführungsform sind die in den Nuten montierbaren oder montierten Leuchteinheiten ausgelegt, im Wesentlichen Licht insbesondere in einem ausgewählten Spektralbereich innerhalb eines Wellenlängenbereichs von etwa 50 nm bis etwa 50 µm (UV bis IR) auszustrahlen. Vorzugsweise strahlen die Leuchteinheiten zumindest 90% der abgestrahlten Lichtleistung mit einer vorbestimmten Wellenlänge oder einem vorbestimmten Wellenlängenspektrum innerhalb eines Bereichs zwischen 50 nm und 50 µm ab. Die Leuchteinheiten können somit dazu ausgebildet sein, elektromagnetische Strahlung mit der vorbestimmten Wellenlänge oder einem vorbestimmten Wellenlängenspektrum abzustrahlen. Hierbei kann es sich insbesondere um Laserlicht und/oder Licht von LEDs handeln. Die vorbestimmte Wellenlänge oder das vorbestimmte Wellenlängenspektrum kann auf den Inhalt des Einwegbehälters abgestimmt sein, insbesondere auf Wellenlängen, welche den Stoffwechsel der darin befindlichen Zellen beeinflussen. Hierbei können die Leuchteinheiten z.B. dazu ausgebildet sein, zumindest 90% der abgestrahlten Lichtleistung bei der vorbestimmten Wellenlänge oder in dem vorbestimmten Wellenlängenspektrum abzustrahlen. Die Leuchteinheiten können auch Lichtquellengruppen bilden, welche bei unterschiedlichen Wellenlängen Licht ausstrahlen und vorzugsweise selektiv angesteuert werden können, um die spektrale Verteilung des in das Behälterinnere eingestrahlten Lichts zu verändern.

In einer Ausführungsform verlaufen eine Vielzahl von Nuten in der Aufnahmebehälterinnenwand ringförmig und/oder parallel zueinander, insbesondere in einer Betriebsstellung der Vorrichtung horizontal. So verlaufen die Nuten in ihrer Längserstreckung zumindest im Bereich von Seitenwandabschnitten der Aufnahmebehälterinnenwand in der Betriebsstellung bzw. -position der Vorrichtung im Wesentlichen horizontal, also insbesondere jeweils in einer Ebene senkrecht zu einer (virtuellen) Zylinderachse von Zylindermantelsegmenten der Seitenwandabschnitte. Der horizontale Verlauf der Nuten erlaubt selbst bei unterschiedlichem Füllstand im Behälterinneren (oder bei unterschiedlich hohen Einwegbehältern eine gezielte Beleuchtung nur im Bereich der Füllung durch Aktivierung von Leuchteinheiten in einzelnen Nuten bis hin zur Füllhöhe und Deaktivierung oberhalb der Füllhöhe. Damit vermeidet man Austrocknung und Einbrennen von Zellmaterial, das oberhalb des Flüssigkeitsspiegels am Einwegbehälter haftet. Zudem kann auch in dieser Variante die Aufnahmebehälterinnenwand abschnittsweise mit den Nuten planar vorgefertigt und anschließend entsprechend der gewünschten Zylinderkrümmung verformt werden. Hierbei ergibt sich der zusätzliche Vorteil, dass die mit den Nuten planar vorgefertigte Aufnahmebehälterinnenwand anschließend rund gebogen werden kann, ohne dass unkontrollierte bzw. eckige Verformungen (z.B. entlang des Verlaufs der Nuten) entstehen.

In einer anderen Ausführungsform verlaufen eine Vielzahl der Nuten geradlinig und/oder parallel zueinander und sind vorzugsweise äquidistant zueinander angeordnet. Dies erlaubt einerseits eine möglichst einfache Herstellung und Montage der Vorrichtung. Andererseits unterstützt es eine möglichst homogene Ausleuchtung des Behälterinnenraums. Auch lokale Inhomogenitäten in der Erwärmung der Aufnahmebehälterinnenwand aufgrund des Betriebes der Leuchteinheiten werden dadurch gering gehalten.

In einer Ausführungsform bildet die Aufnahmebehälterinnenwand zumindest abschnittweise einen Teil einer Zylindermantelfläche und besonders bevorzugt ist sie ganz oder zumindest teilweise abschnittsweise aus Teilen von Zylindermantelflächen zusammengesetzt. Dabei verlaufen die Nuten in einem oder in jedem solchen Teil einer Zylindermantelfläche vorzugsweise parallel zur jeweiligen Zylinderachse. Sie sind also so angeordnet, dass ihre Längserstreckung parallel zur Achsrichtung der jeweiligen Zylindermantelfläche liegt. Dies kann insbesondere fertigungstechnische Vorteile bringen. So können beispielsweise LED-Leuchtbänder sehr einfach innerhalb der geradlinigen Nuten verbaut werden. Auch die Nutabdeckungen können einfach durch im Wesentlichen planare Plättchen (z.B. aus Glas oder Plexiglas) gefertigt und montiert werden, wobei mechanische Spannungen vermieden werden. Es ist auf diese Weise auch relativ einfach möglich, die Aufnahmebehälterinnenwand zunächst planar mit einer Vielzahl parallel verlaufender Nuten zu fertigen, um einzelne Abschnitte danach um eine Achse (Zylinderachse) parallel zum Nutverlauf zu krümmen.

Vorzugsweise umfasst die Aufnahmebehälterinnenwand zumindest teilweise Metall oder sie ist zumindest teilweise aus Metall gebildet, wobei hier als Metall beispielsweise Aluminium und/oder Edelstahl verwendet wird. Dies ist besondere im Hinblick auf die thermische Leitfähigkeit von Metall besonders bevorzugt, insbesondere um die im Betrieb der elektrischen Funktionselemente (z.B. Leuchteinheiten) entstehende Wärme effizient ableiten zu können und/oder um die Temperatur im Innenraum des Aufnahmebehälter effizient regeln zu können.

Insbesondere in diesem Zusammenhang ist es besonders vorteilhaft, wenn die Vorrichtung außerdem eine Temperiereinheit zum Temperieren der Aufnahmebehälterinnenwand umfasst. In einer Ausführungsform weist dazu der Aufnahmebehälter eine Aufnahmebehälteraußenwand auf, die zusammen mit der Aufnahmebehälterinnenwand einen Temperierzwischenraum bildet, welcher durch ein Temperierfluid gefüllt und/oder durchströmt werden kann, um den Behälterinnenraum zu temperieren. Die zumindest eine Aufnahmebehälterinnenwand ist mittels der Temperiereinheit temperierbar. Die Temperiereinheit kann die Aufnahmebehälterinnenwand auf eine vorbestimmbare, insbesondere auf eine einstellbare Temperatur temperieren. Die Temperiereinheit kann insbesondere zum Kühlen der Aufnahmebehälterinnenwand ausgelegt sein. Dabei kann die Temperiereinheit entweder unmittelbar an einer dem Behälterinnenraum abgewandten Außenseite der Aufnahmebehälterinnenwand angeordnet sein, oder zumindest in direktem Wärmeaustausch mit der Aufnahmebehälterinnenwand stehen. Die Aufnahmebehälterinnenwand kann dabei einen hohen Temperaturleitwert aufweisen, kann also z.B. metallisch ausgebildet sein.

Bei einer Anordnung von Leuchteinheiten in den (zum Behälterinnraum hin offenen) Nuten der Aufnahmebehälterinnenwand (welche höchstens durch eine Lichtdurchlässige Nutabdeckung abgedeckt sind) stehen die Leuchteinheiten somit in unmittelbarer Nähe zum Einwegbehälter, um dessen Inhalt effizient beleuchten zu können. Andererseits stehen sie im gut wärmeleitenden Kontakt mit der ebenfalls wärmeleitfähigen Aufnahmebehälterinnenwand. Durch die Nähe zwischen Einwegbehälter und Leuchteinheiten wird eine besonders effiziente Lichteinstrahlung in das Innere des Einwegbehälters mit stark reduzierten Verlusten ermöglicht. Zugleich kann über das Temperieren mit der Temperiereinheit eine negative, zu starke Hitzeentwicklung der Leuchteinheiten vermieden werden, die ansonsten sowohl zu einer Schädigung der Zellkulturen als auch zu einer Beschädigung einer aus Kunststoff ausgebildeten Wand des Einwegbehälters führen könnte.

Damit ist die Vorrichtung besonders geeignet zur Aufnahme von Einwegbehältern, die ein Kulturvolumen für eine mikrobielle Kultur, eine Zellkultur, eine Kultur von Zell- und/oder Gewebsverbänden von pflanzlichen und/oder tierischen Zellen und/oder Hybridformen aufweisen.

Vorzugsweise ist durch die Temperiereinheit eine maximale Kühlleistung von mindestens etwa 5 kW, vorzugsweise mindestens etwa 10 kW, weiter bevorzugt mindestens etwa 15 kW, am meisten bevorzugt mindestens etwa 20 kW erreichbar.

Gemäß einer Ausführungsform sind an zumindest 10%, vorzugsweise zumindest 20%, noch bevorzugt zumindest 30%, am meisten bevorzugt zumindest 40% der Fläche der Innenseite aller lateralen Aufnahmebehälterinnenwände Nuten insbesondere zur Aufnahme von Leuchteinheiten ausgebildet. Dadurch wird die Lichtleistung und/oder die Anzahl von Photonen sowie die Homogenität ihrer Verteilung erhöht, die in den Behälterinnenraum abgestrahlt werden können. Ein Ziel kann hierbei eine weitgehend gleichmäßige Beleuchtung über die Außenflächen des Behälters sein. Die lateralen Aufnahmebehälterinnenwände sind hierbei diejenigen Wände des Aufnahmebehälters, die den Behälterinnenraum in einer lateralen Richtung begrenzen. Dies sind z.B. bei einem im Wesentlichen zylinderförmigen, aufrechtstehenden Aufnahmebehälter alle Wände des Zylindermantels ohne den Zylinderboden und ohne den Zylinderdeckel.

Gemäß einer Ausführungsform steht in einer Betriebsstellung bzw. -position der Vorrichtung der Einwegbehälter in Berührkontakt mit der Innenfläche der Aufnahmebehälterinnenwand (im Bereich zwischen den Nuten) und mit den Leuchteinheiten oder den Nutabdeckungen (im Bereich der Nuten). Dies ermöglicht eine besonders effiziente oder verlustarme Beleuchtung und Temperierung des Inhalts des Einwegbehälters.

Gemäß einer Ausführungsform ist der Aufnahmebehälter dazu ausgebildet, Einwegbehälter mit einem Volumen von zumindest etwa 1 Litern, vorzugsweise zumindest etwa 10 Litern, noch mehr bevorzugt zumindest etwa 50 Litern, weiter bevorzugt zumindest etwa 100 Litern, noch weiter bevorzugt zumindest etwa 200 Litern, besonders bevorzugt zumindest etwa 500 Litern, ganz besonders bevorzugt zumindest etwa 1000 Litern, am meisten bevorzugt zumindest etwa 2000 Litern aufzunehmen. Beispielsweise fasst der Behälterinnenraum ein Volumen im Bereich von etwa 1 Liter bis etwa 5000 Liter, vorzugsweise im Bereich von etwa 50 Liter bis etwa 2000 Liter. Bei vorbekannten Vorrichtungen war es bislang nicht möglich, Einwegbehälter mit solch großen Volumina gleichermaßen effizient zu beleuchten und zu kultivieren. Erst die besonders effiziente Verbindung der Beleuchtung und Kühlung durch die erfindungsgemäße Vorrichtung ermöglicht die hierfür benötigte besonders gleichmäßige Lichtbeaufschlagung und Temperaturkontrolle. Dabei ist zu beachten, dass das von den Leuchteinheiten emittierte Licht unter Umständen zwar nicht vollständig bis zur Mitte des Mediums in dem Einwegbehälter vordringt, aber durch eine Vermischung des Mediums mittels einer Rührvorrichtung trotzdem eine ausreichend gleichmäßige Beleuchtung des Mediums bereitgestellt werden kann. Durch die Möglichkeit der Einbettung der Leuchteinheiten die Nuten der Aufnahmebehälterinnenwand werden außerdem mechanische Belastungen des Einwegbehälters und lokale Temperaturspitzen an Innenkanten des Aufnahmebehälters vermieden. Der Aufnahmebehälter lässt sich außerdem sehr einfach zu zuverlässig reinigen.

Gemäß einer Ausführungsform erfüllt die Vorrichtung die GMP Richtlinien. Die GMP Richtlinien, was abgekürzt für "Good Manufacturing Practice" steht, sind Richtlinien über Anforderungen an Hygiene, Räumlichkeiten, Ausrüstung, Dokumentation und Kontrollen im pharmazeutischen Bereich. Die Vorrichtung kann so ausgebildet sein, dass sie die in den GMP Richtlinien genannten Anforderungen erfüllt, insbesondere die Anforderungen an Sterilität.

Insgesamt bietet die Erfindung zumindest in einigen der beschriebenen Ausführungsformen einige Vorteile sowohl bei der Herstellung als auch im Betrieb. So erlaubt die Einbettung der elektrischen Funktionselemente (insbesondere Leuchteinheiten) in Nuten der Aufnahmebehälterinnenwand eine einfache und zuverlässige Reinigbarkeit des Inneren des Aufnahmebehälters. Außerdem sind die elektrischen Funktionselemente (z.B. LEDs) durch den Einbau in den Nuten und in besonderer Weise durch das Verschließen der Nuten mit Nutabdeckungen selbst bei unachtsamer Handhabung gut geschützt. Gerade bezüglich der Verwendung von Nutabdeckungen erlaubt die Ausbildung von Nutschultern als Auflageflächen für die Nutabdeckungen eine sichere Montage (z.B. durch Verklebung), indem hierdurch sowohl das Anbringen der Nutabdeckungen vereinfacht wird als auch eine hohe Haltbarkeit im Betrieb gewährleistet wird. Aber auch ansonsten ist allein schon die Montage der elektrischen Funktionselemente in den Nuten einfach in der Montage als auch besonders haltbar im Betrieb. Außerdem kann durch eine Vermeidung von direktem Kontakt von LED-Leuchtflächen und Einwegbehältern eine Gefahr der Entstehung von Hot-Spots (Temperaturspitzen) verringert werden. Auch die Verkabelung lässt sich zumindest im Bereich der Anschlüsse zu den elektrischen Funktionselementen (z.B. LED) innerhalb der Nuten verlegen und vorzugsweise zusammen mit den elektrischen Funktionselementen eingießen, wodurch die Anschlüsse sehr einfach und zuverlässig gegen mechanische oder äußere chemische Einflüsse geschützt werden können.

Die Erfindung wird nachfolgend anhand von in Figuren gezeigten Ausführungsbeispielen näher beschrieben. Gleiche Bezugszeichen bezeichnen gleiche oder ähnliche Bauteile der Ausführungsformen. Einzelne Merkmale der Ausführungsformen können mit anderen Ausführungsformen kombiniert werden. Es zeigen:
Figur 1 in einer perspektivischen Darstellung eine Vorrichtung zum Aufnehmen eines Einwegbeutels;
Figur 2 in einer perspektivischen Darstellung eine vertikale Schnittansicht durch eine Vorrichtung zum Aufnehmen eines Einwegbeutels;
Figur 3 in einer schematischen Darstellung einen Querschnitt durch eine Aufnahmebehälterinnenwand mit einer in einer Nut untergebrachten Leuchteinheit;
Figur 4 in einer schematischen Darstellung eine Aufnahmebehälterinnenwand mit einer Vielzahl paralleler Nuten und teilweise montierten Leuchteinheiten;
Figur 5 in einer perspektivischen Darstellung eine schematische Ansicht einer geöffneten Vorrichtung zum Aufnehmen eines Einwegbeutels;
Figur 6 in einer perspektivischen Darstellung eine schematische Ansicht einer weiteren geöffneten Vorrichtung zum Aufnehmen eines Einwegbeutels.

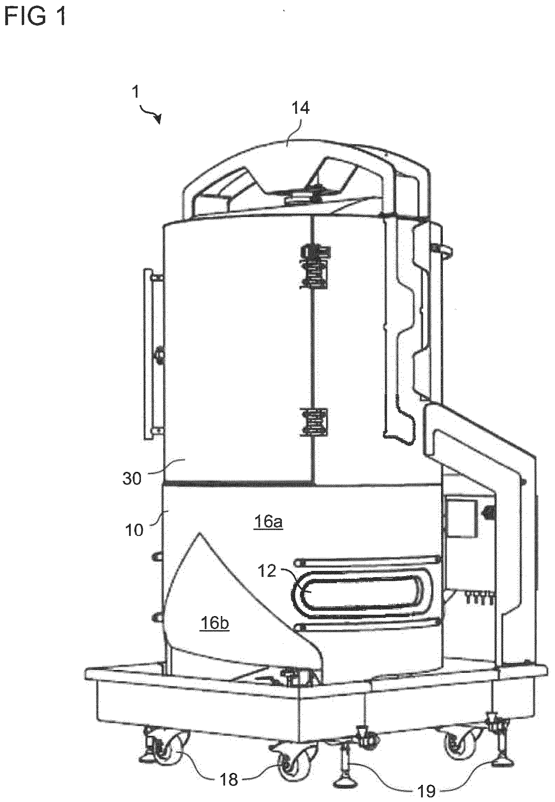

Figur 1 zeigt in einer perspektivischen Darstellung eine Vorrichtung 1 zum Aufnehmen eines Einwegbeutels als Einwegbehälter. Die in den Figuren gezeigte Vorrichtung 1 kann als ein Bestandteil eines Systems zum Aufnehmen eines Einwegbeutels ausgebildet sein. Die Vorrichtung 1 weist einen Aufnahmebehälter 10 auf, der im Wesentlichen die Form eines vertikal angeordneten Zylinders aufweist, d.h. dessen Zylinderachse im Wesentlichen vertikal angeordnet ist. Der Aufnahmebehälter 10 weist einen Behälterinnenraum auf, in den ein Einwegbeutel eingebracht werden kann, der zum Beispiel ein biologisches Medium beinhalten kann. Das biologische Medium im Einwegbeutel wird im Behälterinnenraum des Aufnahmebehälters 10 über einen vorbestimmbaren Zeitraum gelagert und/oder beleuchtet. Während sich der Einwegbeutel mit dem biologischen Medium im Inneren des Aufnahmebehälters 10 befindet, können sich unterschiedliche Reaktionen mit oder an dem biologischen Medium ereignen. Somit kann die Vorrichtung 1 auch als Bioreaktor ausgebildet sein.

Zur Beobachtung des biologischen Mediums sind in einem Seitenwandabschnitt 16a ein oder mehrere Sichtfenster 12 ausgebildet, durch das bzw. die von außen durch die Aufnahmebehälterwand hindurch in den Behälterinnenraum des Aufnahmebehälters 10 hineingeblickt werden kann, um das biologische Medium zu beobachten. Um den Behälterinnenraum zugänglich zu machen, weist die Vorrichtung vorzugsweise außerdem eine Behältertür 30 auf. Die Behältertür 30 erstreckt sich in der Breite, also in horizontaler Richtung, beispielsweise in etwa über ein Zylindersegment (von beispielsweise ca. 100°) des Aufnahmebehälters 10 und umfasst dabei vorzugsweise auch einen Teil einer Aufnahmebehälterinnenwand, in der später noch gezeigte Nuten ausgebildet sind.

Bei geöffneter Behältertür 30 ist ein Zugriff auf den Behälterinnenraum des Aufnahmebehälters 10 möglich. Durch die Türöffnung kann zum Beispiel der Einwegbeutel von einer seitlichen Richtung her, also im Wesentlichen in einer horizontalen Bewegungsrichtung, in den Behälterinnenraum des Aufnahmebehälters 10 eingeführt werden.

Die Vorrichtung 1 ist auf Rollen 18 rollbar gelagert, auf denen die Vorrichtung rollend durch einen Raum geschoben werden kann. Zusätzlich zu den Rollen 18 kann die Vorrichtung 1 am unteren Ende Fixierfüße 19 aufweisen, die zum Fixieren und korrektem Ausrichten der Vorrichtung 1 z.B. auf unebenen Böden dienen.

In der dargestellten Ausführungsform ist der Aufnahmebehälter 10 nach oben offen ausgebildet. Anstelle eines Zylinderdeckels (oder in einem nicht dargestellten Zylinderdeckel) weist der Aufnahmebehälter 10 eine Rühröffnung auf. Oberhalb des nach oben offenen Aufnahmebehälters 10 ist eine Rührvorrichtung 14 ausgebildet, über die ein Rührstab durch die Rühröffnung hindurch mit dem Einwegbeutel so verbunden werden kann, dass das Innere des Einwegbeutels durchmischt werden kann. Der Rührstab kann im Inneren des Einwegbeutels angeordnet sein und über eine Kopplung bzw. Kupplung mit der Rührvorrichtung 14 verbunden werden. Die Rührvorrichtung 14 ist zentral über dem Aufnahmebehälter 10 ausgebildet und wird von einer Trägerbrücke getragen, die auf einem oberen Rand des Aufnahmebehälters 10 an einander gegenüberliegenden Seitenwandabschnitten des Aufnahmebehälters 10 aufliegt.

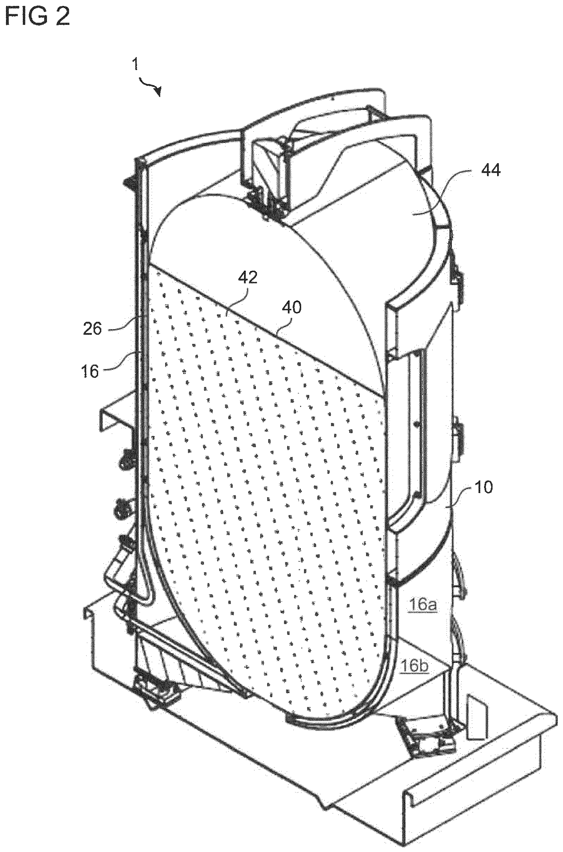

Figur 2 zeigt in einer perspektivischen Darstellung eine Ansicht eines vertikalen Schnitts durch die Vorrichtung 1, wobei auch in dieser Ansicht die in Nuten eingebrachten Leuchteinheiten noch nicht explizit visualisiert sind. Sie zeigen zum Behälterinnenraum hin, welcher in dieser Darstellung mit einem biologischen Medium 42 gefüllt ist. In Figur 2 gezeigt ist z.B. ein Einwegbeutel 44 als Einwegbehälter, genauer ein Schnitt durch diesen Einwegbeutel 44, der im Behälterinnenraum des Aufnahmebehälters 10 angeordnet ist. Im Behälterinnenraum des Aufnahmebehälters 10 und zugleich auch im Inneren des Einwegbeutels 44 ist das biologische Medium 42 angeordnet, das bis auf Höhe einer vorbestimmten Füllstandhöhe 40 gefüllt ist. Das biologische Medium 42 erstreckt sich vom Boden des Aufnahmebehälters 10 bis hin zur Füllstandhöhe 40 und füllt somit insbesondere das gesamte Innenvolumen des Aufnahmebehälters 10 bis hin zur Füllstandhöhe 40, abzüglich des Volumens der Wände des Einwegbeutels 44. Der Einwegbeutel 44 wird durch eine Behälterwand 16 des Aufnahmebehälters 10 in Form gehalten, die insbesondere einen Bodenabschnitt 16b und den Seitenwandabschnitt 16a umfasst und sich vom (teilweise abgerundeten) Bodenabschnitt 16b des Aufnahmebehälters 10 bis über die Füllstandhöhe 40 hinaus nach oben erstrecken kann. Die Behälterwand 16 bildet oder umfasst dabei insbesondere eine Aufnahmebehälterinnenwand 26, deren Nutstruktur später noch genauer gezeigt ist.

In der dargestellten Ausführungsform ist die Behälterwand 16 aus mehreren Abschnitten zusammengesetzt, welche insbesondere den Seitenwandabschnitt 16a und einen Bodenabschnitt 16b umfassen. Vorzugsweise ist jeder der Abschnitte im Wesentlichen als Segment eines Zylindermantels geformt. Dabei verläuft beispielsweise in der dargestellten Ausführungsform eine (virtuelle) Zylinderachse der Zylindermantelform des Seitenwandabschnitts 16a im Wesentlichen vertikal, während eine (virtuelle) Zylinderachse der Zylindermantelform des Bodenabschnitts 16b im Wesentlichen horizontal verläuft. Besonders bevorzugt verlaufen auch die später noch genauer dargestellten Nuten in den Aufnahmebehälterinnenwänden parallel zur zugehörigen (virtuellen) Zylinderachse des entsprechenden Abschnitts. Damit können auch die Leuchteinheiten und eventuelle Nutabdeckungen einfach geradlinig oder sogar im Wesentlichen planar gefertigt und montiert werden.

Die Behälterwände 16 des Aufnahmebehälters 10 sind zumindest teilweise als eine Temperierhohlwand ausgebildet, in der ein (in den Figuren nicht gezeigtes) Temperiermedium fließt. Das Temperiermedium kann auf einen Niedrigdruck kleiner als 0,5 bar geregelt werden, oder auf einen Druck bis etwa 6 bar geregelt werden. Die Temperierhohlwand kann sich über die gesamte Aufnamebehälterwände 16 einschließlich des Bodenabschnitts 16b und des Seitenwandabschnitts 16a vom Behälterboden nach oben bis über die Füllstandhöhe 40 hinaus bis zu einer vorbestimmbaren Temperierhöhe erstrecken. Die Temperierhöhe kann im Wesentlichen 1 cm bis 20 cm vertikal oberhalb der Füllstandhöhe 40 angeordnet sein. Die mit den Nuten versehene Aufnahmebehälterinnenwand 26 kann dabei als Innenwand der Temperierhohlwand ausgebildet sein oder an die Innenwand der Temperierhohlwand angeordnet und thermisch damit verbunden sein. Mit anderen Worten kann die Aufnahmebehälterinnenwand 26 entweder separate von der Temperierhohlwand gefertigt und danach mit der Temperierhohlwand verbunden werden oder direkt als Teil der Temperierhohlwand hergestellt werden.

Das biologische Medium 42 kann (über die Beutelwand des Einwegbeutels 44) in Wärmekontakt zur Aufnahmebehälterinnenwand 26 (und damit direkt oder indirekt zur Temperierhohlwand) stehen. Über das Temperiermedium kann somit das biologische Medium 42 auf eine vorbestimmbare Temperatur geregelt werden. Die Vorrichtung 1 kann insbesondere dazu ausgebildet und vorgesehen sein, den Behälterinnenraum auf eine vorbestimmbare Solltemperatur von etwa 0°C bis etwa 80°C, bevorzugt von etwa 20°C bis etwa 40°C zu temperieren. Die Temperierhohlwand umgibt den Behälterinnenraum des Aufnahmebehälters 10 bis über die Füllstandhöhe 40 hinaus beinahe vollständig. "Beinahe vollständig umgeben" bedeutet in den in den Figuren gezeigten Ausführungsbeispielen, dass die Temperierhohlwand den Behälterinnenraum bis zur Temperierhöhe hin vollständig bis auf diejenigen Positionen umgibt, an denen die ggf. vorhandenen Sichtfenster 12 angeordnet sind. Selbst die Behältertür 30 kann in einer Ausführungsform mit einer Temperierhohlwand und/oder mit einer Nuten aufweisenden Aufnahmebehälterinnenwand ausgebildet sein.

Alternativ oder zusätzlich zur Temperierhohlwand ist es auch möglich, dass in zumindest einigen der Nuten in der Aufnahmebehälterinnenwand Temperierelemente (z.B. elektrische Heiz- und/oder Kühlelemente) aufgenommen sind. In diesem Fall dienen somit nicht alle Nuten zur Aufnahme von Leuchteinheiten, sondern einige davon können der Aufnahme von (insbesondere elektrischen) Temperierelementen dienen.

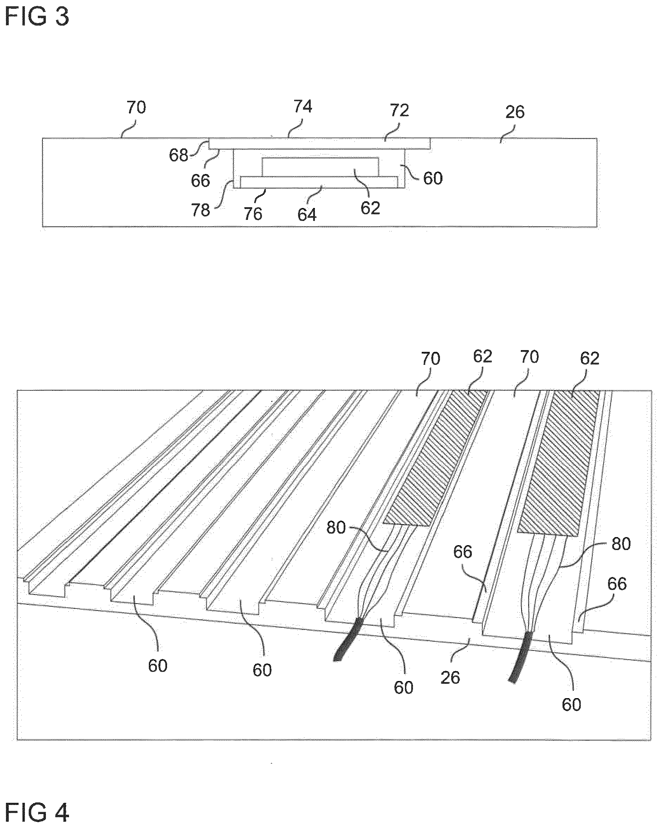

Fig. 3 zeigt in einer schematischen Darstellung einen Querschnitt durch eine Aufnahmebehälterinnenwand 26 senkrecht zu einer Längserstreckung von Nuten mit einer in einer Nut 60 untergebrachten Leuchteinheit 62. Die Aufnahmebehälterinnenwand 26 ist im Schnitt von Fig. 3 planar dargestellt, obwohl sie in der Anwendung vorzugsweise zylindrisch gekrümmt ist, insbesondere mit einer Zylinderachse parallel zur Längsrichtung der Nut 60 (also senkrecht zur Zeichnungsebene von Fig. 3). In einer bevorzugten Ausführungsform kann die Aufnahmebehälterinnenwand 26 aber zunächst planar gefertigt werden, also mit den Nuten 60 versehen werden, bevor sie dann für die Herstellung oder Zusammensetzung der Vorrichtung 1 vorzugsweise zylinderförmig gekrümmt wird. Da der Krümmungsradius der zylinderförmigen Krümmung aber vorzugsweise wesentlich größer ist als die lateralen Dimensionen der Nuten, also die Nutenbreiten, werden sich bei dieser Umformung die schematisch dargestellten geometrischen Verhältnisse nur geringfügig verändern. Aber auch diese geringfügigen Änderungen können bei der Herstellung berücksichtigt werden, um alle nachfolgend noch beschriebenen Komponenten problemlos in die Nuten einfügen zu können. Besonderes bevorzugt wird die Aufnahmebehälterinnenwand 26 mittels Biegen und/oder Walzen und/oder Fräsen und/oder Bohren und/oder 3D-Drucken, besonders bevorzugt aus Metall gefertigt.

In der in Fig. 3 dargestellten Ausführungsform umfasst die Leuchteinheit 62 eine Vielzahl von Lichtquellen, die auf einem gemeinsamen Träger 64 angeordnet sind. Die Lichtquellen können beispielsweise einzelne LEDs sein, die auf dem Täger 64 angeordnet sind. In der dargestellten Ausführungsform weisen die Nuten 60 beidseitig eine Nutschulter 66 auf, die um eine erste Stufe 68 relativ zu einer zwischen den Nuten gebildeten Innenfläche 70 der Aufnahmebehälterinnenwand 26 zurückversetzt ist. Diese Nutschulter 68 dient dabei als Auflagefläche für eine Nutabdeckung 72, welche die Nut zum Behälterinnenraum hin derart verschließt, dass eine Innenfläche 74 der Nutabdeckung 72 zusammen mit der Innenfläche 70 der Aufnahmebehälterinnwand 26 eine stetige Fläche, insbesondere ohne Kanten bildet. Zumindest am Übergang zwischen der Innenfläche 74 der Nutabdeckung 72 und der Innenfläche 70 der Aufnahmebehälterinnenwand 26 tritt vorzugsweise keine Kante, vorzugsweise zumindest aber keine konvexe Kante auf. Die Höhe der ersten Stufe 68 liegt dabei vorzugsweise in einem Bereich von etwa 0,5 mm bis etwa 5 mm, vorzugsweise in einem Bereich von etwa 1 mm bis etwa 3 mm. Sie entspricht im Wesentlichen einer Dicke der Nutabdeckung 72 insoweit als dadurch die stetige Innenfläche erreicht wird. Soweit die Nutabdeckung 72 mit der Nutschulter 66 verklebt ist, ist die erst Schulter 68 vorzugsweise um die Dicke der Klebstoffschicht größer als die Dicke der Nutabdeckung 72. Vorzugsweise liegt die Breite der jeweiligen Nutschulter 66 in einem Bereich von etwa 1 mm bis etwa 5 mm, vorzugsweise in Bereich von etwa 1 mm bis etwa 3 mm.

In der dargestellten Ausführungsform ist außerdem ein Nutengrund 76 als vorzugsweise planare Bodenfläche des zentralen Nutkanals um eine zweite Stufe 78 relativ zu den beiden Nutschultern 66 zurückversetzt. Die Leuchteinheit 60, vorzugsweise zusammen mit dem Träger 64, ist am Nutengrund 76 angebracht. Dabei liegt die Höhe der zweiten Stufe 78 (also die Tiefe des Nutengrunds relativ zu den Nutschultern 66) in einem Bereich von etwa 2 mm bis etwa 15 mm, vorzugsweise in einem Bereich von etwa 2 mm bis etwa 10 mm, noch mehr bevorzugt in einem Bereich von etwa 3 mm bis etwa 5 mm. Die Höhe der zweiten Stufe 78 ist insbesondere zumindest so groß wie oder vorzugsweise größer als eine Höhe der Leuchteinheit 62 (inklusive Träger 64). Ein verbleibender Hohlraum innerhalb der Nut 60 ist vorzugsweise durch ein Gussmaterial ausgefüllt, so dass die Leuchteinheit 62 in der Nut 60 zumindest teilweise durch das Gussmaterial bedeckt oder eingegossen ist. Eine Breite des zentralen Nutkanals (senkrecht zu dessen Längserstreckung), also insbesondere eine Breite des Nutengrunds 76 ist beispielsweise in einem Bereich von etwa 5 mm bis etwa 30 mm, vorzugsweise in einem Bereich von etwa 10 mm bis etwa 20 mm. Damit lassen sich beispielsweise LED-Streifen gut in den Nuten verlegen.

Fig. 4 zeigt in einer schematischen Darstellung eine Aufnahmebehälterinnenwand 26 mit einer Vielzahl paralleler, insbesondere äquidistanter Nuten 60 und teilweise montierten Leuchteinheiten. Diese Darstellung ist wiederum insofern nur schematisch als eine eventuelle zylindrische Krümmung hier nicht explizit dargestellt ist. Die Nuten 60 sind ähnlich zur Darstellung in Fig. 3 mit Nutschultern 66 ausgestattet. In einigen der Nuten 60 sind bereits Leuchteinheiten 62 mit zugehörigen Verkabelungen 80 montiert. Bevor die Nuten 60 mit Nutabdeckungen versehen werden, werden die Leuchteinheiten 62, vorzugsweise mit den in den Nuten 60 verlaufenden Abschnitten der Verkabelungen 80, mittels eines Gussmaterials eingegossen. Die (vergossenen) Nuten werden dann mit Nutabdeckungen verschlossen, welche insbesondere eine kontinuierliche Fläche mit den Innenflächen 70 der Aufnahmebehälterinnenwand 26 bilden.

Grundsätzlich ist es bevorzugt, wenn Abstände benachbarter Nuten 60 (Abstände zwischen den einander zugewandten Nutschultern benachbarter Nuten) zumindest teilweise nicht größer als das fünffache, vorzugsweise nicht größer als das dreifache, am meisten bevorzugte nicht größer als das zweifache der Breite der Nuten (einschließlich ihrer Nutschultern) sind. Damit lässt sich eine möglichst gleichmäßige Beleuchtung erreichen, wenn die Nuten (zumindest teilweise) mit Leuchteinheiten ausgestattet werden.

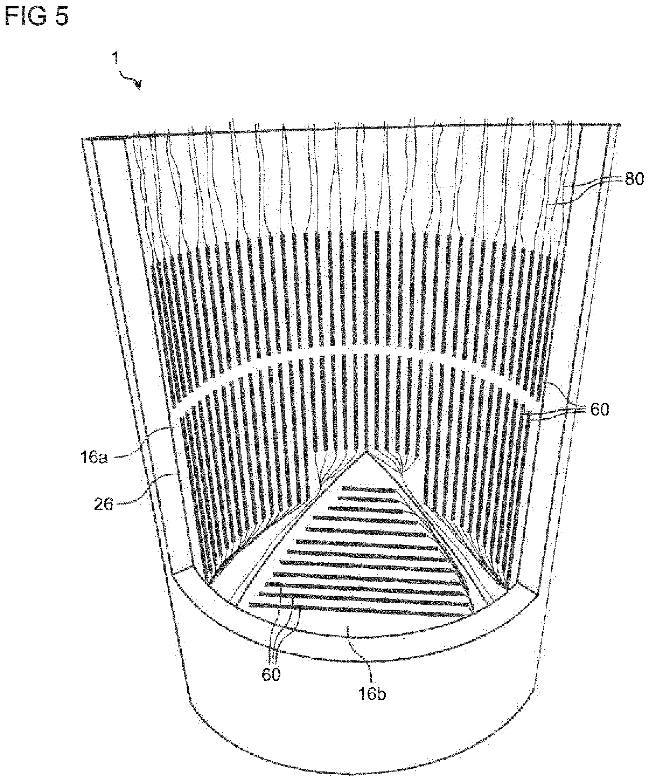

Fig. 5 zeigt in einer perspektivischen Darstellung eine schematische Ansicht einer geöffneten Vorrichtung 1 zum Aufnehmen eines Einwegbeutels. Wie in dieser Darstellung schematisch gezeigt, kann die Aufnahmegehälterinnenwand 26 aus mehreren Abschnitten zusammengesetzt sein, welche insbesondere einen Seitenwandabschnitt 16a und einen Bodenabschnitt 16b des Aufnahmebehälters umfassen. Vorzugsweise sind alle Abschnitte der Aufnahmebehälterinnenwand 26 als Zylindermantelsegmente derart geformt, dass eine (virtuelle) Zylinderachse der Zylindermantelsegmente der Seitenwandabschnitte 16a in der Betriebsstellung bzw. -position der Vorrichtung 1 im Wesentlichen vertikal steht, während eine (virtuelle) Zylinderachse der Zylindermantelsegmente der Bodenabschnitte 16b in der Betriebsstellung bzw. -position der Vorrichtung 1 im Wesentlichen horizontal liegt. Diese Form der Wandabschnitte erlaubt eine einfache Herstellung bei gleichzeitiger Vermeidung von stark konkaven Ecken im Inneren des Aufnahmebehälters.

Insbesondere ist es bevorzugt, wenn die Nuten 60 in den Abschnitten der Aufnahmebehälterinnenwand 26 im Wesentlichen parallel zur jeweiligen (virtuellen) Zylinderachse des entsprechenden Wandabschnitts verlaufen. Damit verlaufen die Nuten 60 in den Seitenwandabschnitten 16a im Wesentlichen vertikal, während die Nuten 60 in den Bodenabschnitten 16b im Wesentlichen horizontal verlaufen. Dadurch verlaufen die Nuten 60 trotz der Krümmung der Aufnahmebehälterinnenwand 26 immer geradlinig. Dies vereinfacht einerseits die Montage der Leuchteinheiten in den Nuten 60. Andererseits begünstigt es aber auch eine möglichst homogene Lichtverteilung. Auch die Herstellung und Montage entsprechend geradlinig verlaufenden, vorzugsweise sogar planar fertigbarer, transparenter Nutabdeckungen wird damit begünstigt. Schließlich ist auch die Herstellung der Aufnahmebehälterinnenwand in dieser Geometrie vergleichsweise einfach. Insbesondere kann die Aufnahmebehälterinnenwand abschnittsweise mit den Nuten planar vorgefertigt und anschließend entsprechend der gewünschten Zylinderkrümmung verformt werden.

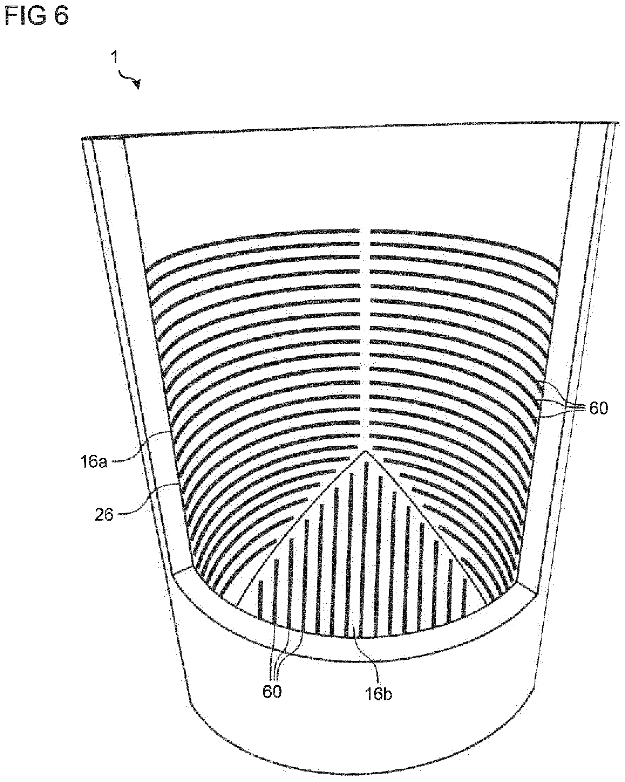

Eine alternative und besonders bevorzugte Anordnung der Nuten 60 ist in Fig. 6 schematisch veranschaulicht. So verlaufen die Nuten 60 in ihrer Längserstreckung zumindest im Bereich von Seitenwandabschnitten 16a der Aufnahmebehälterinnenwand 26 in der Betriebsstellung bzw. -position der Vorrichtung 1 im Wesentlichen horizontal, also insbesondere jeweils in einer Ebene senkrecht zu einer (virtuellen) Zylinderachse von Zylindermantelsegmenten der Seitenwandabschnitte 16a. Der horizontale Verlauf der Nuten erlaubt selbst bei unterschiedlichem Füllstand im Behälterinneren (oder bei unterschiedlich hohen Einwegbehältern eine gezielte Beleuchtung nur im Bereich der Füllung, also bis hin zur Füllhöhe. Damit vermeidet man Austrocknung und Einbrennen von Zellmaterial, das oberhalb des Flüssigkeitsspiegels am Einwegbehälter haftet. Zudem kann auch in dieser Variante die Aufnahmebehälterinnenwand abschnittsweise mit den Nuten planar vorgefertigt und anschließend entsprechend der gewünschten Zylinderkrümmung verformt werden. Hierbei ergibt sich der zusätzliche Vorteil, dass die mit den Nuten planar vorgefertigte Aufnahmebehälterinnenwand anschließend rund gebogen werden kann, ohne dass unkontrollierte bzw. eckige Verformungen (z.B. entlang des Verlaufs der Nuten) entstehen.

## Patentansprüche

1. Vorrichtung (1) zur Aufnahme eines Einwegbehälters (44), umfassend:
- einen Aufnahmebehälter (10) mit einer Aufnahmebehälterinnenwand (16), die einen Behälterinnenraum des Aufnahmebehälters (10) zum Aufnehmen des Einwegbehälters (44) begrenzt wobei in der Aufnahmebehälterinnenwand eine Vielzahl von Nuten zur Aufnahme elektrischer Funktionselemente vorgesehen sind.

2. Vorrichtung nach Anspruch 1, wobei in der Vielzahl von Nuten zumindest teilweise Leuchteinheiten, insbesondere LEDs, zum Beleuchten des Behälterninnenraums montiert sind, wobei vorzugsweise die Leuchteinheiten in den Nuten zumindest teilweise durch ein Gussmaterial bedeckt oder eingegossen sind.

3. Vorrichtung nach Anspruch 2, wobei die Nuten zum Behälterinnenraum hin mittels Nutabdeckungen derart verschlossen sind, dass Innenflächen der Nutabdeckungen zusammen mit zwischen den Nuten gebildeten Innenflächen der Aufnahmebehälterinnwand stetige Flächen bilden.

4. Vorrichtung nach Anspruch 3, wobei zwischen den Leuchteinheiten und der jeweiligen Nutabdeckung ein Luftspalt gebildet ist.

5. Vorrichtung nach Anspruch 3 oder 4, wobei jede der Vielzahl von Nuten einen beidseitig zweistufigen Querschnitt senkrecht zu einer Längsachse der jeweiligen Nut derart aufweist, dass beiderseits eines zentralen Nutkanals jeweils eine Auflagefläche um eine erste Stufe relativ zur Innenfläche der Aufnahmebehälterinnenwand und ein Nutengrund des zentralen Nutkanals um eine zweite Stufe relativ zu den beiden Auflageflächen zurückversetzt sind.

6. Vorrichtung nach Anspruch 5, wobei eine Höhe der ersten Stufe in einem Bereich von etwa 0,5 mm bis etwa 5 mm, vorzugsweise in einem Bereich von etwa 1 mm bis etwa 3 mm liegt; und/oder
wobei die Höhe der zweiten Stufe in einem Bereich von etwa 2 mm bis etwa 15 mm, vorzugsweise in einem Bereich von etwa 2 mm bis etwa 10 mm, noch mehr bevorzugt in einem Bereich von etwa 3 mm bis etwa 5 mm liegt; und/oder
wobei eine Breite der jeweiligen Auflagefläche in einem Bereich von etwa 1 mm bis etwa 5 mm, vorzugsweise in Bereich von etwa 1 mm bis etwa 3 mm liegt.

7. Vorrichtung nach einem der Ansprüche 2 bis 6, wobei die in den Nuten montierbaren oder montierten Leuchteinheiten ausgelegt sind, im Wesentlichen Licht innerhalb eines Wellenlängenbereichs von etwa 50 nm bis etwa 50 µm auszustrahlen.

8. Vorrichtung nach einem der vorangegangenen Ansprüche, wobei eine Vielzahl von Nuten in der Aufnahmebehälterinnenwand (16) ringförmig und/oder parallel zueinander, insbesondere in einer Betriebsstellung der Vorrichtung horizontal verlaufend, angeordnet sind.

9. Vorrichtung nach einem der vorangegangenen Ansprüche, wobei eine Vielzahl der Nuten geradlinig und/oder parallel zueinander verlaufen und vorzugsweise äquidistant zueinander angeordnet sind.

10. Vorrichtung nach einem der vorangegangenen Ansprüche, wobei die Aufnahmebehälterinnenwand (16) zumindest abschnittweise einen Teil einer Zylindermantelfläche bildet oder abschnittsweise aus Teilen von Zylindermantelflächen zusammengesetzt ist, und wobei die Nuten in einem oder in jedem solchen Teil einer Zylindermantelfläche parallel zur jeweiligen Zylinderachse angeordnet sind.

11. Vorrichtung nach einem der vorangegangenen Ansprüche, wobei der Behälterinnenraum ein Volumen im Bereich von etwa 1 Liter bis etwa 5000 Liter, vorzugsweise im Bereich von etwa 50 Liter bis etwa 2000 Liter fasst.

12. Vorrichtung nach einem der vorangegangenen Ansprüche, wobei die Aufnahmebehälterinnenwand zumindest teilweise Metall umfasst bzw. aus Metall gebildet ist.

13. Vorrichtung nach Anspruch einem der vorangegangenen Ansprüche, welche außerdem eine Temperiereinheit zum Temperieren der Aufnahmebehälterinnenwand (16) umfasst.

14. Vorrichtung nach Anspruch 13, wobei durch die Temperiereinheit eine maximale Kühlleistung von mindestens etwa 5 kW, vorzugsweise mindestens etwa 10 kW, weiter bevorzugt mindestens etwa 15 kW, am meisten bevorzugt mindestens etwa 20 kW erreichbar ist.

15. Verwendung einer Vorrichtung gemäß einem der Ansprüche 1 bis 14 zur Prozessierung von phototrophen Organismen.

## Claims

1. Device (1) for receiving a single-use container (44), comprising:
- a receiving container (10) having a receiving container inner wall (16) that delimits a container interior of the receiving container (10) for receiving the single-use container (44), wherein a plurality of grooves for receiving electrical functional elements are provided in the receiving container inner wall.

2. Device according to claim 1, wherein illuminating units, in particular LEDs, for illuminating the container interior are at least partially mounted in the plurality of grooves, wherein preferably the illuminating units in the grooves are at least partially covered or encapsulated by an encapsulating material.

3. Device according to claim 2, wherein the grooves are closed toward the container interior by means of groove covers such that inner surfaces of the groove covers together with inner surfaces of the receiving container inner wall formed between the grooves form continuous surfaces.

4. Device according to claim 3, wherein an air gap is formed between the illuminating units and the respective groove cover.

5. Device according to claim 3 or 4, wherein each of the plurality of grooves has a cross-section, perpendicular to a longitudinal axis of the respective groove, featuring two steps at each side such that, on each side of a central groove channel, a bearing surface is set back by a first step relative to the inner surface of the receiving container inner wall and a groove bottom of the central groove channel is set back by a second step relative to the two bearing surfaces.

6. Device according to claim 5, wherein a height of the first step lies in a range from approximately 0.5 mm to approximately 5 mm, preferably in a range from approximately 1 mm to approximately 3 mm; and/or
wherein the height of the second step lies in a range from approximately 2 mm to approximately 15 mm, preferably in a range from approximately 2 mm to approximately 10 mm, more preferably in a range from approximately 3 mm to approximately 5 mm; and/or
wherein a width of the respective bearing surface lies in a range from approximately 1 mm to approximately 5 mm, preferably in a range from approximately 1 mm to approximately 3 mm.

7. Device according to any one of claims 2 to 6, wherein the illuminating units that can be or are mounted in the grooves are designed to emit light substantially within a wavelength range of from approximately 50 nm to approximately 50 µm.

8. Device according to any one of the preceding claims, wherein a plurality of grooves in the receiving container inner wall (16) are arranged annularly and/or parallel to one another, in particular extending horizontally in an operating position of the device.

9. Device according to any one of the preceding claims, wherein a plurality of the grooves extend rectilinearly and/or parallel to one another and are preferably arranged equidistantly from one another.

10. Device according to any one of the preceding claims, wherein the receiving container inner wall (16) at least in some sections forms part of a cylinder casing surface or in some sections is composed of parts of cylinder casing surfaces, and wherein the grooves in one or in every such part of a cylinder casing surface are arranged parallel to the respective cylinder axis.

11. Device according to any one of the preceding claims, wherein the container interior holds a volume in the range from approximately 1 liter to approximately 5000 liters, preferably in the range from approximately 50 liters to approximately 2000 liters.

12. Device according to any one of the preceding claims, wherein the receiving container inner wall at least partially comprises metal or is formed of metal.

13. Device according to any one of the preceding claims, which further comprises a temperature control unit for controlling the temperature of the receiving container inner wall (16).

14. Device according to claim 13, wherein a maximum cooling capacity of at least approximately 5 kW, preferably at least approximately 10 kW, more preferably at least approximately 15 kW, most preferably at least approximately 20 kW, can be achieved by the temperature control unit.

15. Use of a device according to any one of claims 1 to 14 for processing phototrophic organisms.

## Revendications

1. Dispositif (1) de réception d'un contenant à usage unique (44), comprenant :
- un contenant de réception (10) avec une paroi intérieure de contenant de réception (16) qui délimite un espace intérieur de contenant du contenant de réception (10) pour la réception du contenant à usage unique (44), dans lequel une pluralité de rainures sont prévues dans la paroi intérieure de contenant de réception pour la réception d'éléments fonctionnels électriques.

2. Dispositif selon la revendication 1, dans lequel des unités d'éclairage, en particulier des DEL, sont montées au moins partiellement dans la pluralité de rainures pour l'éclairage de l'espace intérieur de contenant, dans lequel les unités d'éclairage dans les rainures sont de préférence au moins partiellement recouvertes par un matériau coulé, ou coulées.

3. Dispositif selon la revendication 2, dans lequel en allant vers l'espace intérieur de contenant les rainures sont fermées au moyen de couvercles de rainure de sorte que des surfaces intérieures des couvercles de rainure ensemble avec des surfaces intérieures, formées entre les rainures, de la paroi intérieure de contenant de réception forment des surfaces continues.

4. Dispositif selon la revendication 3, dans lequel un intervalle d'air est formé entre les unités d'éclairage et le couvercle de rainure respectif.

5. Dispositif selon la revendication 3 ou 4, dans lequel chacune de la pluralité de rainures présente perpendiculairement à un axe longitudinal de la rainure respective une coupe transversale à deux niveaux sur les deux côtés telle que des deux côtés d'un canal de rainure central soient respectivement reportés en arrière une surface d'application à hauteur d'un premier niveau par rapport à la surface intérieure de la paroi intérieure de contenant de réception et un fond de rainure du canal de rainure central à hauteur d'un second niveau par rapport aux deux surfaces d'application.

6. Dispositif selon la revendication 5, dans lequel une hauteur du premier niveau se situe dans une plage d'environ 0,5 mm à environ 5 mm, de préférence dans une plage d'environ 1 mm à environ 3 mm; et/ou
dans lequel la hauteur du second niveau se situe dans une plage d'environ 2 mm à environ 15 mm, de préférence dans une plage d'environ 2 mm à environ 10 mm, davantage de préférence encore dans une plage d'environ 3 mm à environ 5 mm; et/ou
dans lequel une largeur de la surface d'application respective se situe dans une plage d'environ 1 mm à environ 5 mm, de préférence dans une plage d'environ 1 mm à environ 3 mm.

7. Dispositif selon l'une des revendications 2 à 6, dans lequel les unités d'éclairage pouvant être montées ou déjà montées dans les rainures sont conçues pour essentiellement émettre une lumière à l'intérieur d'une plage de longueur d'onde d'environ 50 nm à environ 50 µm.

8. Dispositif selon l'une des revendications précédentes, dans lequel une pluralité de rainures dans la paroi intérieure de contenant de réception (16) sont agencées de façon annulaire et/ou parallèlement les unes aux autres, en particulier se déroulant horizontalement dans une position de fonctionnement du dispositif.

9. Dispositif selon l'une des revendications précédentes, dans lequel une pluralité des rainures se déroulent de façon rectiligne et/ou parallèlement les unes aux autres, et sont de préférence agencées à équidistance les unes des autres.

10. Dispositif selon l'une des revendications précédentes, dans lequel la paroi intérieure de contenant de réception (16) forme au moins par sections une partie d'une surface d'enveloppe de cylindre ou est composée par sections de parties de surfaces d'enveloppe de cylindre, et dans lequel les rainures sont agencées dans une ou dans chaque telle partie d'une surface d'enveloppe de cylindre parallèlement à l'axe de cylindre respectif.

11. Dispositif selon l'une des revendications précédentes, dans lequel l'espace intérieur de contenant contient un volume dans la plage d'environ 1 litre à environ 5 000 litres, de préférence dans la plage d'environ 50 litres à environ 2000 litres.

12. Dispositif selon l'une des revendications précédentes, dans lequel la paroi intérieure de contenant de réception, au moins partiellement, comprend du métal ou est formé à partir de métal.

13. Dispositif selon l'une des revendications précédentes, lequel comprend en outre une unité d'équilibrage de température pour l'équilibrage de température de la paroi intérieure de contenant de réception (16).

14. Dispositif selon la revendication 13, dans lequel une capacité de refroidissement maximale d'au moins environ 5 kW, de préférence d'au moins environ 10 kW, davantage de préférence d'au moins environ 15 kW, le plus préférablement d'au moins environ 20 kW peut être obtenue par l'unité d'équilibrage de température.

15. Utilisation d'un dispositif selon l'une des revendications 1 à 14 pour la culture d'organismes phototropes.
